# EUROPEAN PATENT APPLICATION

(11) **EP 4 190 891 A1**
(43) Date of publication of application: **07.06.2023**
(21) Application number: 21898046.4
(22) Date of filing: 25.11.2021
(51) Int. Cl.: C12N 5/00, C12M 1/00, C12M 3/00, C12N 1/04

(54) **METHOD FOR MANUFACTURING CELL MASS**

(30) Priority: 26.11.2020 JP 2020195707
(71) Applicant: Tosoh Corporation, Yamaguchi 746-8501 (JP)
(72) Inventor: KUNO Goshi, Yokkaichi-shi, Mie 510-8540 (JP)
(74) Representative: Novagraaf International SA
(86) International application number: PCT/JP2021/043257
(87) International publication number: WO 2022/114074

(57) **Abstract**

A method for manufacturing a cell mass adherently cultured on a cell culture substrate, in which the cell culture substrate comprises two regions (A) and (B), and the method comprising steps (1) to (3): (A) an island-shaped region having a cell proliferation property and an area of 0.001 to 1 mm²; and (B) a region adjacent to the region (A) and having no cell proliferation properties, (1) a step of preparing a cell suspension comprising a mesenchymal cell and an ectoderm-derived cell; (2) a step of bringing the cell suspension into contact with the cell culture substrate, and randomly adhering the two types of cells to the region (A), and in which, on the contact surface between the cell suspension and the cell culture substrate, a total number of cells of the two types of cells is 100 to 100,000 cells/cm²; and (3) a step of culturing the cell adhered to the region (A) and forming a cell mass comprising the two types of cells.

## Description

### Technical Field

The present invention relates to a method for manufacturing a cell mass, the method enabling a cell mass comprising two or more types of cells comprising mesenchymal cells and ectoderm-derived cells, which is a cell mass comprising at least two portions: a portion having a larger content of ectoderm-derived cells and a portion having a smaller content of the cells (larger content of mesenchymal cells), to be efficiently manufactured and stably transported.

### Background Art

In recent years, there have been active attempts to regenerate damaged organs or the like by transplanting ex vivo cultured cells into human bodies. As a cell source for regenerative medicine, in addition to a method of utilizing pluripotent stem cells such as iPS cells, a method of transplanting cell masses prepared from epithelial cells and mesenchymal cells called organ primordium has been reported.

As a method for manufacturing an organ primordium, a method of arranging two types of cell masses: a cell mass of epithelial cells and a cell mass of mesenchymal cells, adjacent to each other in a collagen gel has been reported (for example, refer to Patent Literature 1). However, in this method, it is necessary to prepare each organ primordium individually one by one in a gel, and there is a problem of poor mass productivity of organ primordia.

As another method of preparing an organ primordium, a method in which a cell culture substrate having a U-bottomed shape to which cells are not adhered is used, epithelial cells and mesenchymal cells that are mixed are cultured, and cell masses in which epithelial cells and mesenchymal cells aggregate together are spontaneously formed is known (for example, refer to Patent Literature 2). Although this method is excellent in mass productivity of organ primordia, since the cell masses are present in a suspended state, there are problems of the cell masses being easily moved during a culture operation and transport of organ primordia due to shaking of a medium, and organ primordia being easily damaged.

### Citation List

### Patent Literature

[Patent Literature 1] JP5932671
[Patent Literature 2] JP6425319

### Summary of Invention

### Technical Problem

An object of the present invention is to provide a method for manufacturing a cell mass, the method enabling a cell mass comprising two or more types of cells, which is a cell mass comprising at least two portions: a portion having a larger content of ectoderm-derived cells and a portion having a smaller content of the cells (larger content of mesenchymal cells) when the cells are adhered to a substrate, to be efficiently manufactured and stably transported.

### Solution to Problem

The inventors conducted extensive studies in view of the above points and as a result, found that the above problems can be solved by adherently culturing two or more types of cells on a cell culture substrate having pattered cell adhesion regions, and completed the present invention.

Specifically, the present invention is as follows:
<1> A method for manufacturing a cell mass adherently cultured on a cell culture substrate in which the cell culture substrate comprises the following two regions (A) and (B), and the method comprising the following steps (1) to (3):
   (A) an island-shaped region having a cell proliferation property and an area of 0.001 to 1 mm²; and
   (B) a region adjacent to the region (A) and having no cell proliferation properties,

   (1) a step of preparing a cell suspension comprising a mesenchymal cell and an ectoderm-derived cell;
   (2) a step of bringing the cell suspension prepared in the step (1) into contact with the cell culture substrate, and randomly adhering the mesenchymal cell and the ectoderm-derived cell to the region (A), and in which, on the contact surface between the cell suspension and the cell culture substrate, a total number of cells of the mesenchymal cell and the ectoderm-derived cell is 100 to 100,000 cells per unit area; and
   (3) a step of culturing the cell adhered to the region (A) and forming a cell mass comprising the mesenchymal cell and the ectoderm-derived cell.
<2> The manufacturing method according to <1>,
   wherein a ratio between the number of mesenchymal cell and the number of ectoderm-derived cell in the cell suspension is 1:10 to 10:1.
<3> The method for manufacturing a cell mass according to <1> or <2>,
   wherein, in the step (3), the cell mass comprises a portion having a larger content of the mesenchymal cell than the ectoderm-derived cell and a portion having a larger content of the ectoderm-derived cell than the mesenchymal cell, and a ratio between the largest cross-sectional area of a portion having a larger content of the mesenchymal cell than the ectoderm-derived cell in the substrate in-plane direction and the largest cross-sectional area of a portion having a larger proportion of the ectoderm-derived cell than the mesenchymal cell in the substrate in-plane direction is 2:1 to 1:20.
<4> The method for manufacturing a cell mass according to any one of claims <1> to <3>, further comprising the following step (4):
   (4) a step of culturing the cell mass formed in the step (3) and forming an organ primordium.
<5> The method for manufacturing a cell mass according to <4>,
   wherein Versican is present in the organ primordium.
<6> A differentiation induction kit for hair follicle primordia comprising a cell culture substrate having the following two regions (A) and (B):
   (A) a circular region having a cell proliferation property and an area of 0.001 to 1 mm², and
   (B) a region adjacent to the region (A) and having no cell proliferation properties.
<7> The differentiation induction kit for hair follicle primordia according to <6>, further comprising a medium in which a mesenchymal cell and an epithelial cell have proliferating properties.

### Advantageous Effects of Invention

The present invention can provide a method for manufacturing a cell mass, method enabling a cell mass comprising two or more types of cells comprising mesenchymal cells and ectoderm-derived cells, which is a cell mass comprising at least two portions: a portion having a larger content of ectoderm-derived cells and a portion having a smaller content of the cells (larger content of mesenchymal cells), to be efficiently manufactured and stably transported.

In addition, in the method for manufacturing a cell mass of the present invention, when a portion having a larger content of ectoderm-derived cells and a portion having a larger content of mesenchymal cells are formed, a tissue structure similar to that in vivo is generated, and the manufactured cell mass can differentiate into mature state cells that have the ability to regenerate in the human body.

### Brief Description of Drawings

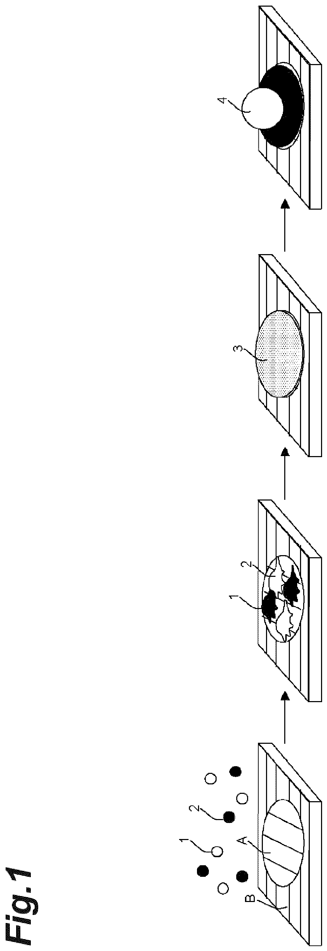

FIG. 1 is a schematic diagram illustrating a method for manufacturing a cell mass of the present invention.

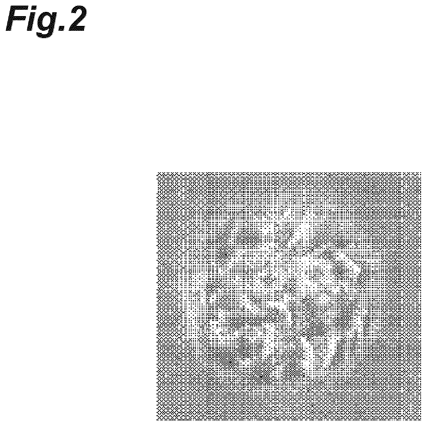

FIG. 2 is a phase contrast microscopic image of a cell mass on the 1st day of culture of Example 1.

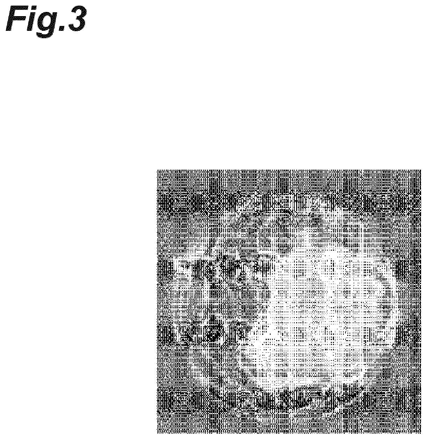

FIG. 3 is a phase contrast microscopic image of a cell mass on the 6th day of culture of Example 1.

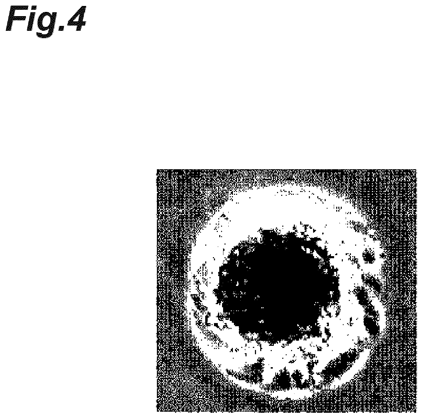

FIG. 4 is a phase contrast microscopic image of a cell mass on the 11th day of culture of Example 1.

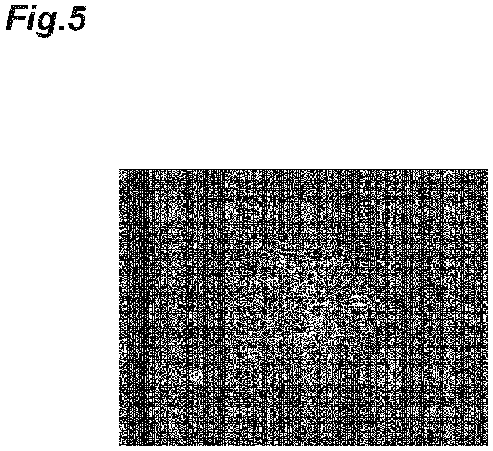

FIG. 5 is a phase contrast microscopic image of a cell mass on the 1st day of culture of Example 3.

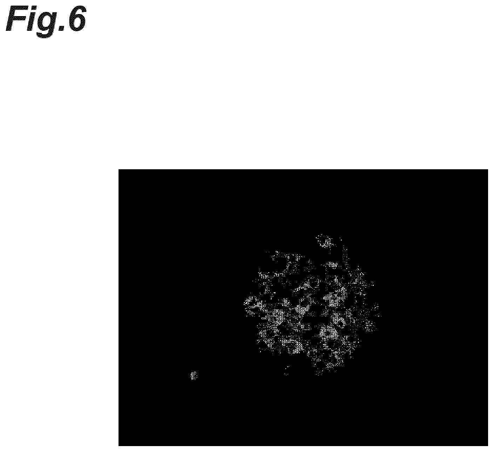

FIG. 6 is a fluorescence microscopic image of a cell mass on the 1st day of culture of Example 3.

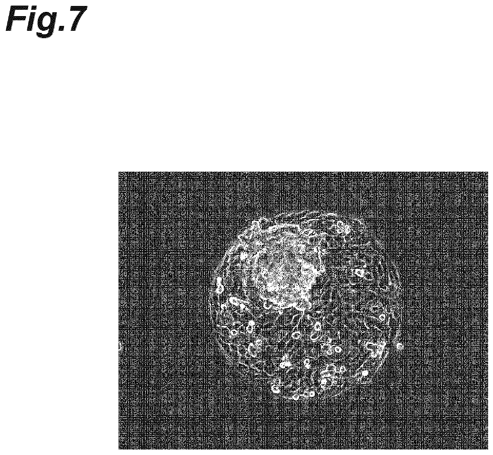

FIG. 7 is a phase contrast microscopic image of a cell mass on the 7th day of culture of Example 3.

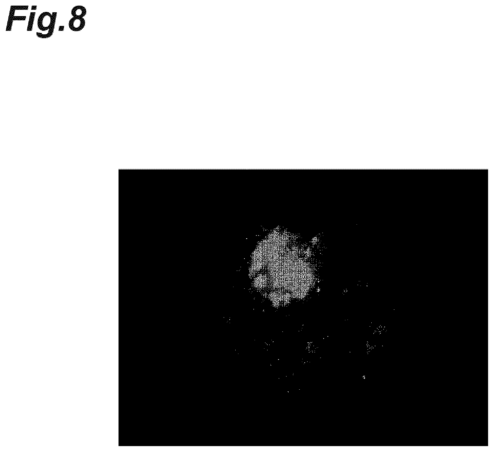

FIG. 8 is a fluorescence microscopic image of a cell mass on the 7th day of culture of Example 3.

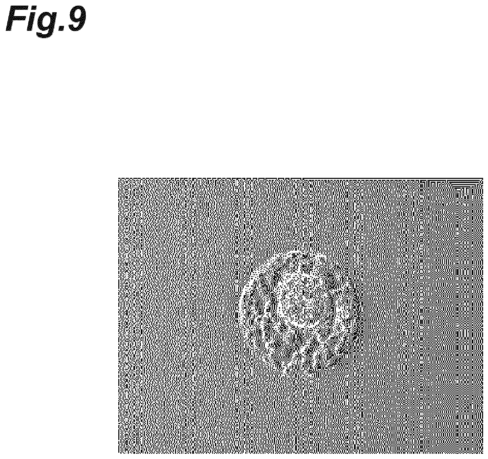

FIG. 9 is a phase contrast microscopic image of a cell mass on the 7th day of culture of Example 4.

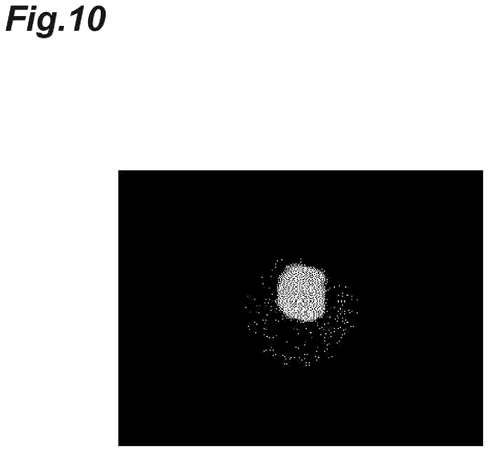

FIG. 10 is a fluorescence microscopic image of a cell mass on the 7th day of culture of Example 4.

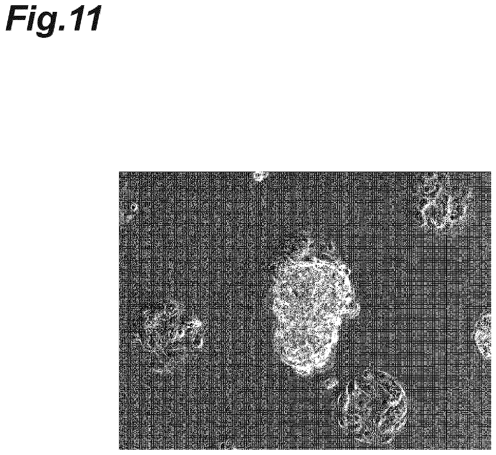

FIG. 11 is a phase contrast microscopic image of a cell mass on the 7th day of culture of Example 5.

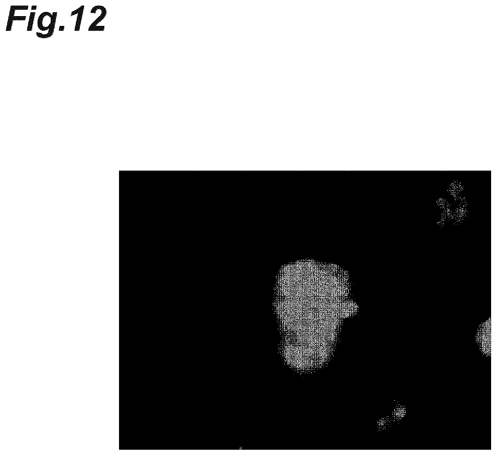

FIG. 12 is a fluorescence microscopic image of a cell mass on the 7th day of culture of Example 5.

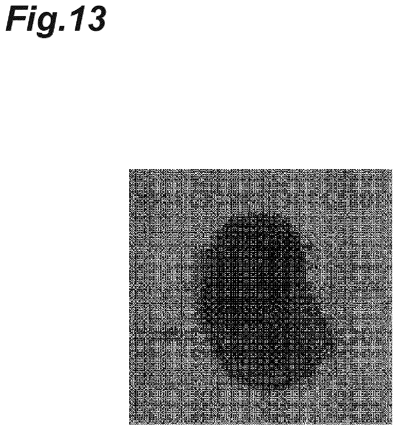

FIG. 13 is a phase contrast microscopic image of a cell mass on the 6th day of culture of Comparative Example 4.

### Description of Embodiments

Hereinafter, a form for implementing the present invention (hereinafter simply referred to as "the present embodiment") will be described in detail. The following present embodiment is only an example for describing the present invention, and the present invention is not limited to the following content. The present invention can be appropriately modified and implemented within the spirit and scope of the invention.

In this specification, "cell mass" refers to a three-dimensional agglomerate of cells formed by aggregating a plurality of cells. In addition, "organ primordium" is a cell mass formed from epithelial cells and mesenchymal cells, and indicates a cell mass that can regenerate tissues and organs by transplantation into living bodies.

In addition, in this specification, "co-culture" means culture of a mixture of two or more types of cells.

In addition, in this specification, "stimulus responsiveness" means that the structure is changed or the degree of hydrophilicity/hydrophobicity is changed according to an external stimulus. Here, in this specification, "external stimulus" refers to a mechanical stimulus such as ultrasonic waves, vibration, and convection, an electromagnetic stimulus such as light, electricity, and magnetism, and a thermodynamic stimulus such as heating and cooling, other than a stimulus caused by a biological reaction such as an enzymatic reaction.

In addition, in this specification, "temperature responsiveness" means that the degree of hydrophilicity/hydrophobicity is changed when the temperature changes. Further, the boundary temperature at which the degree of hydrophilicity/hydrophobicity changes is referred to as a "response temperature."

In addition, in this specification, "cell adhesion" means ease of adhesion to a cell culture substrate at a temperature at which cells are cultured. "Having cell adhesion" means that cells can adhere to a substrate or a cell culture substrate directly or via an organism-derived substance at a culture temperature. In addition, "having no cell adhesion" means that cells cannot adhere to a substrate or a cell culture substrate at a culture temperature.

In addition, in this specification, "cell proliferation properties" refers to ease of cell proliferation at a culture temperature, and "having cell proliferation properties" means that cells adhere to a substrate or a cell culture substrate directly or via an organism-derived substance at a culture temperature, and can additionally proliferate. In addition, "having no cell proliferation properties" means that cells cannot adhere to a substrate or a cell culture substrate at a culture temperature or do adhere thereto but cannot proliferate. Further, "better cell proliferation properties" means that cells proliferate into more cells when compared in the same culture period.

In addition, in this specification, "organism-derived substance" is a substance present in the body of an organism, and may be a natural substance or a substance artificially synthesized by a gene modification technology or the like, or may be a substance chemically synthesized based on the organism-derived substance. The organism-derived substance is not particularly limited, and may be, for example, nucleic acids, proteins, and polysaccharides which are basic materials constituting living bodies, nucleotides, nucleosides, amino acids, or various sugars which are constituents thereof, or lipids, vitamins, or hormones.

One aspect of the present invention relates to a method for manufacturing a cell mass adherently cultured on a cell culture substrate in which the cell culture substrate has the following two regions (A) and (B), the method comprising:
the following steps (1) to (3):
(A) an island-shaped region having cell proliferation properties and an area of 0.001 to 1 mm², and
(B) a region adjacent to the region (A) and having no cell proliferation properties,
   (1) a step of preparing a cell suspension comprising mesenchymal cells and ectoderm-derived cells;
   (2) a step of bringing the cell suspension prepared in the step (1) into contact with the cell culture substrate, and randomly adhering the mesenchymal cells and the ectoderm-derived cells to the region (A), and in which, on the contact surface between the cell suspension and the cell culture substrate, a total number of cells of the mesenchymal cells and the ectoderm-derived cells is 100 to 100,000 cells per unit area; and
   (3) a step of culturing the cells adhered to the region (A) and forming a cell mass comprising the mesenchymal cells and the ectoderm-derived cells.

The cell culture substrate used in the present invention has the following two regions (A) and (B).
(A) an island-shaped region having cell proliferation properties and an area of 0.001 to 1 mm², and
(B) a region adjacent to the region (A) and having no cell proliferation properties.

The region (A) has cell proliferation properties. When it has cell proliferation properties, the cell culture substrate of the present invention can culture cells to manufacture a cell mass. Without cell proliferation properties, cells cannot be cultured to form a cell mass.

The region (A) is an island-shaped region having an area of 0.001 to 1 mm². Since the region (A) is an island-shaped region having an area of 0.001 to 1 mm², when two or more types of cells are adherently cultured, one type of cells can spontaneously aggregate to form a portion having a larger content of the cells. If the area is less than 0.001 mm², cells cannot be uniformly co-cultured in the entire region (A). In addition, if the area exceeds 1 mm², when two or more types of cells are co-cultured, all cells become uniformly dispersed to form a cell mass, and it is not possible to manufacture a cell mass having a portion in which one type of cells spontaneously aggregate and the content of the cells is large. The area is preferably 0.005 to 0.5 mm², the area is more preferably 0.01 to 0.5 mm², the area is particularly preferably 0.015 to 0.25 mm², and the area is most preferably 0.02 to 0.2 mm² because it is suitable to form a cell mass having the ability to regenerate in the human body such as an organ primordium. In addition, in culturing hair follicle primordia, the area is preferably 0.001 to 0.2 mm², the area is more preferably 0.001 to 0.1 mm², the area is particularly preferably 0.001 to 0.05 mm², and the area is most preferably 0.005 to 0.05 mm².

In addition, the standard deviation/average area of the area of the region (A) is preferably 80% or less, more preferably 50% or less, particularly preferably 20% or less, and most preferably 5% or less because it is suitable to manufacture cell masses having a uniform size and shape. The shape of the region (A) is not particularly limited, and can be appropriately set according to the shape of a desired cell mass, and examples thereof include a circle, an ellipse, a polygon, and a closed shape formed with irregular straight lines or curves. In addition, a circle, an ellipse, or a polygon is preferable, a circle, an ellipse, or a rectangle is more preferable, a circle, an ellipse, or a square is particularly preferable, and a circle or an ellipse is most preferable because it is suitable to manufacture a cell mass having a shape close to a sphere.

The region (B) is adjacent to the region (A) and does not have cell proliferation properties. Since the region (B) is a region adjacent to the region (A) and having no cell proliferation properties, it is possible to proliferate cells only in the region (A) when cells are cultured. If the region (B) is not adjacent to the region (A) or has cell proliferation properties, since cells spread around the region (A) when cells are cultured, it is not possible to manufacture a cell mass having a portion in which one type of cells spontaneously aggregate and the content of the cells is large. In addition, it is preferable that the region (B) have neither cell proliferation properties nor have cell adhesion so that it is suitable to make the size and shape of the manufactured cell masses uniform.

The shape of the region (B) is not limited, except that it is adjacent to the region (A), and the region (B) is preferably adjacent to the length of 20% or more, more preferably 50% or more, particularly preferably 80% or more of the boundary line with the region (A), and all the surrounding of the region (A) is most preferably the region (B) so that it is suitable to manufacture cell masses having a uniform size and shape.

The area ratio between the region (A) and the region (B) is not particularly limited, and the area of the region (A) with respect to the entire substrate is preferably 10% or more, more preferably 30% or more, particularly preferably 50% or more, and most preferably 70% or more so that it is suitable to increase the amount of cell masses that can be manufactured per unit area of the culture substrate. In addition, the area of the region (B) with respect to the entire substrate is preferably 20% or more, more preferably 40% or more, particularly preferably 60% or more, and most preferably 80% or more so that it is suitable to provide a sufficient distance between the plurality of regions (A) and to prevent cell masses in the plurality of regions (A) from fusing to form a non-uniform shape.

The cell culture substrate used in the present invention has a layer formed of a hydrophilic polymer on the surface so that it is suitable to form cell masses with a uniform size, and the region (A) is preferably a region in which a part of the layer formed of the hydrophilic polymer is decomposed or modified by any of a plasma treatment, a UV treatment, and a corona discharge treatment or a combination thereof. When the cell culture substrate has a layer formed of a hydrophilic polymer on the surface, adsorption of proteins that contribute to substrate-cell adhesion in the region (B) can be inhibited to form a region having no cell adhesion or cell proliferation properties. In addition, when a part of the layer formed of a hydrophilic polymer is decomposed or modified, it is possible to impart cell adhesion or cell proliferation properties to the region (A). In addition, the region (A) is more preferably a plasma treatment region because it is suitable to improve cell adhesion and cell proliferation properties of the region (A) and form a cell mass in a short time.

In addition, as another method of preparing the cell culture substrate used in the present invention, a method of applying a substance that promotes or inhibits cell adhesion to only a partial region on the cell culture substrate by a photolithographic method, an inkjet method, a screen printing method or the like may be exemplified.

The layer thickness of the layer formed of the hydrophilic polymer is preferably 10 nm or more, more preferably 50 nm or more, particularly preferably 100 nm or more, and most preferably 500 nm or more so that it is suitable to form the region (B) as a region having no cell adhesion or cell proliferation properties. In addition, the layer thickness is preferably 1,000 nm or less, more preferably 500 nm or less, particularly preferably 100 nm or less, and most preferably 50 nm or less so that it is suitable to make the region (A) a region having cell adhesion and cell proliferation properties.

As a method of forming the layer formed of the hydrophilic polymer, at least one of a method of forming a chemical bond and a method of physical interaction can be used. Examples of methods of forming a chemical bond include a method of forming a reactive functional group such as UV emission, electron beam emission, gamma ray emission, a plasma treatment, and a corona treatment. In addition, it is possible to perform a cross-linking reaction on the surface of the substrate according to an organic reaction using ions and radicals as a reaction source. Regarding a method according to a physical interaction, methods such as coating, brush coating, dip coating, spin coating, bar coding, flow coating, spray coating, roll coating, air knife coating, blade coating, gravure coating, micro gravure coating, and slot die coating, using a matrix having excellent compatibility with a desired hydrophilic polymer as a coating material, can be used.

The type of the hydrophilic polymer is not particularly limited, and those having a polar group such as a hydroxy group, an amino group, and a polyethylene glycol group and those having a betaine structure and a zwitterion structure such as a phosphorylcholine group may be exemplified. A hydroxy group, a phosphorylcholine group, or a polyethylene glycol group is preferable, a hydroxy group or a phosphorylcholine group is more preferable, and a phosphorylcholine group is particularly preferable so that it is suitable to form the region (B) as a region having no cell adhesion and cell proliferation properties. As commercial products, for example, BIOSURFINE-AWP (commercially available from Toyo Gosei Co., Ltd.), Lipidure-CM5206 (commercially available from NOF Corporation) and the like can be suitably used.

The hydrophilic polymer is preferably a random copolymer or block copolymer having both hydrophilic monomer units and hydrophobic/reactive monomer units and more preferably a random copolymer having both hydrophilic monomer units and hydrophobic/reactive monomer units so that it is suitable to prevent the hydrophilic polymer from eluting from the cell culture substrate and reduce the influence on quality due to the polymer mixing into cell aggregates and cell masses. In addition, regarding the composition ratio of the copolymer, the proportion of the hydrophilic monomer unit is preferably 30 wt% or more, more preferably 40 wt% or more, particularly preferably 50 wt% or more, and most preferably 60 wt% or more because it is suitable to form the region (B) as a region having no cell adhesion and cell proliferation properties. In addition, the proportion of the hydrophilic monomer unit is preferably 80 wt% or less, more preferably 50 wt% or less, particularly preferably 30 wt% or less, and most preferably 10 wt% or less so that it is suitable to prevent the hydrophilic polymer from eluting.

The hydrophilic monomer unit is not particularly limited, except that it is hydrophilic, and examples thereof include those having an amino group such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl]acrylamide; those having betaine such as N-(3-sulfopropyl)-N-methacryloyloxyethyl-N,N-dimethylammonium betaine, and N-methacryloyloxyethyl-N,N-dimethylammonium-α-N-methylcarboxy betaine; those having a polyethylene glycol group or a methoxyethyl group such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethylacrylamide, furfuryl acrylate, furfuryl methacrylate, tetrahydrofurfuryl acrylate, and tetrahydrofurfuryl methacrylate; those having an acrylate group such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethylacrylamide; and those having a phosphorylcholine group such as 2-methacryloyloxyethylphosphorylcholine, 2-acryloyloxyethylphosphorylcholine, 3-(meth)acryloyloxypropylphosphorylcholine, 4-(meth)acryloyloxybutylphosphorylcholine, 6-(meth)acryloyloxyhexylphosphorylcholine, 10-(meth)acryloyloxydecylphosphorylcholine, ω-(meth)acryloyl(poly)oxyethylenephosphorylcholine, 2-acrylamide ethylphosphorylcholine, 3-acrylamide propylphosphorylcholine, 4-acrylamide butylphosphorylcholine, 6-acrylamide hexylphosphorylcholine, 10-acrylamide decylphosphorylcholine, and ω-(meth)acrylamide (poly)oxyethylene phosphorylcholine.

The hydrophobic monomer unit is not particularly limited, except that it is hydrophobic, and examples thereof include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, and n-tetradecyl methacrylate. The reactive monomer unit is preferably a monomer unit having UV reactivity because it is possible to immobilize the hydrophilic polymer on a substrate according to a short-time treatment, and examples thereof include 4-azidophenyl acrylate, 4-azidophenyl methacrylate, 2-((4-azidobenzoyl)oxy)ethyl acrylate, and 2-((4-azidobenzoyl)oxy)ethyl methacrylate.

The cell culture substrate used in the present invention may have stimulus responsiveness. When the cell culture substrate has stimulus responsiveness, it is possible to separate the cell mass from the cell culture substrate with an external stimulus, it is possible to reduce damage to cells, and collect cells. The stimulus responsiveness is not particularly limited as long as the cell mass can be separated with an external stimulus, and examples thereof include temperature responsiveness, photoresponsiveness, pH responsiveness, magnetic responsiveness, electric field responsiveness, and mechanical stimulus responsiveness, and any of temperature responsiveness, photoresponsiveness, pH responsiveness, and mechanical stimulus responsiveness, or a combination of a plurality thereof is preferable, any of temperature responsiveness, photoresponsiveness, and mechanical stimulus responsiveness or a combination of a plurality thereof is more preferable, any of temperature responsiveness and mechanical stimulus responsiveness or a combination of a plurality thereof is particularly preferable, and temperature responsiveness is most preferable.

In addition, the temperature responsiveness is not particularly limited, and the response temperature is preferably 50°C or lower and more preferably 35°C or lower because cells can be cultured at a temperature close to the body temperature when cells are cultured on the cell culture substrate, and the response temperature is particularly preferably 25°C or lower and most preferably 15°C or lower because it is suitable to prevent cells from being separated when an operation such as replacement of a medium during culturing is performed. In addition, the lower limit value of the response temperature is preferably 0°C or higher, more preferably 1°C or higher, particularly preferably 3°C or higher, and most preferably 4°C or higher so that it is possible to form a cell mass by performing a cooling operation at a temperature at which cells are not damaged. As a method of imparting the temperature responsiveness to the cell culture substrate, a method of providing a layer formed of a temperature-responsive polymer on the surface of the substrate is preferable because it is excellent in mass productivity of the cell culture substrate. The type of the polymer is not particularly limited, and a block copolymer immobilized on a substrate by a physical action such as a hydrophobic interaction, a copolymer immobilized on a substrate via a reactive group such as an azide group, a polymer immobilized on a substrate by applying a monomer to the substrate and performing electron beam polymerization or radical polymerization on the substrate and the like can be suitably used.

The type of the temperature-responsive polymer is not particularly limited, and examples of monomer units for imparting temperature responsiveness include (meth)acrylamide compounds such as acrylamide and methacrylamide; N-alkyl-substituted (meth)acrylamide derivatives such as N,N-diethylacrylamide, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-t-butylacrylamide, N-ethoxyethylacrylamide, N-ethoxyethylmethacrylamide, N-tetrahydrofurfurylacrylamide, and N-tetrahydrofurfurylmethacrylamide; N,N-dialkyl substituted (meth)acrylamide derivatives such as N,N-dimethyl(meth)acrylamide, N,N-ethylmethylacrylamide, and N,N-diethylacrylamide; (meth)acrylamide derivatives having a cyclic group such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine; vinyl ethers such as methyl vinyl ether; and proline derivatives such as N-proline methyl ester acrylamide, and N,N-diethylacrylamide, N-n-propyl acrylamide, N-isopropylacrylamide, N-n-propylmethacrylamide, N-ethoxyethyl acrylamide, N-tetrahydrofurfurylacrylamide, and N-tetrahydrofurfurylmethacrylamide are preferable, N-n-propyl acrylamide and N-isopropylacrylamide are more preferable, and N-isopropylacrylamide is particularly preferable so that it is suitable for setting the response temperature to 0 to 50°C. In addition, if a culture medium at room temperature is used when replacing the culture medium in the culture operation, N-n-propyl acrylamide and N-proline methyl ester acrylamide are preferable so that it is suitable for setting the response temperature of the block copolymer to a temperature lower than room temperature.

In the present invention, the ratio of temperature-responsive constituent units comprised in the temperature-responsive polymer is preferably 70 wt% or more, more preferably 80 wt% or more, particularly preferably 90 wt% or more, and most preferably 92 wt% or more so that it is suitable for rapidly performing a step of forming a cell mass from cell masses.

The temperature-responsive polymer is preferably a random copolymer or block copolymer having both temperature-responsive monomer units and hydrophobic monomer units and more preferably a block copolymer having both hydrophilic monomer units and hydrophobic monomer units so that it is suitable to prevent the temperature-responsive polymer from eluting from the cell culture substrate and reduce the influence on quality due the polymer mixed in cell aggregates and cell masses. As the hydrophobic monomer unit, those as in the above hydrophilic polymer can be suitably used

In the present invention, the temperature-responsive polymer may also comprise monomer units for controlling the response temperature. The monomer units may comprise, for example, hydrophilic or hydrophobic monomer units, and are not particularly limited, and examples thereof include those having an amino group such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl]acrylamide; those having betaine such as N-(3-sulfopropyl)-N-methacryloyloxyethyl-N,N-dimethylammonium betaine, and N-methacryloyloxyethyl-N,N-dimethylammonium-α-N-methylcarboxy betaine; those having a polyethylene glycol group or a methoxyethyl group such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethylacrylamide, furfuryl acrylate, furfuryl methacrylate, tetrahydrofurfuryl acrylate, and tetrahydrofurfuryl methacrylate; those having an acrylate group such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethylacrylamide; and those having a phosphorylcholine group such as 2-methacryloyloxyethylphosphorylcholine, 2-acryloyloxyethylphosphorylcholine, 3-(meth)acryloyloxypropylphosphorylcholine, 4-(meth)acryloyloxybutylphosphorylcholine, 6-(meth)acryloyloxyhexylphosphorylcholine, 10-(meth)acryloyloxydecylphosphorylcholine, ω-(meth)acryloyl(poly)oxyethylenephosphorylcholine, 2-acrylamide ethylphosphorylcholine, 3-acrylamide propylphosphorylcholine, 4-acrylamide butylphosphorylcholine, 6-acrylamide hexylphosphorylcholine, 10-acrylamide decylphosphorylcholine, and ω-(meth)acrylamide (poly)oxyethylene phosphorylcholine.

The molecular weight of the temperature-responsive polymer in the present invention is not particularly limited, and the number average molecular weight is preferably 1,000 to 1,000,000, more preferably 2,000 to 500,000, particularly preferably 5,000 to 300,000, and most preferably 10,000 to 200,000 so that it is suitable to increase the strength of the temperature-responsive polymer.

In the present invention, the content of a component having a number average molecular weight of 5,000 or less comprised in the temperature-responsive polymer is preferably 50% or less, more preferably 30% or less, particularly preferably 10% or less, and most preferably 5% or less so that it is suitable to prevent the temperature-responsive polymer from being mixed into cell masses. In addition, the content of a component having a number average molecular weight of 10,000 or less is preferably 50% or less, more preferably 30% or less, particularly preferably 10% or less, and most preferably 5% or less. In addition, the content of a component having a number average molecular weight of 30,000 or less is preferably 50% or less, more preferably 30% or less, particularly preferably 10% or less, and most preferably 5% or less. The content of components having a specific molecular weight or less comprised in the block copolymer can be measured by gel permeation chromatography.

The hydrophilic/temperature-responsive polymer in the present invention may comprise, as necessary, a chain transfer agent, a polymerization initiator, a polymerization inhibitor and the like. The chain transfer agent is not particularly limited, and those generally used can be suitably used, and examples thereof include dithiobenzoate, trithiocarbonate, 4-cyano-4-[(dodecylsulfonylthiocarbonyl)sulfonyl]pentanoic acid, 2-cyanopropan-2-yl N-methyl-N-(pyridin-4-yl)carbamodithioate, and methyl 2-methylpropionate(4-pyridinyl)carbamodithioate. In addition, the polymerization initiator is not particularly limited, and those generally used can be suitably used, and examples thereof include azobisisobutyronitrile, 1,1'-azobis(cyclohexanecarbonitrile), di-tert-butyl peroxide, tert-butyl hydroperoxide, hydrogen peroxide, potassium peroxydisulfate, benzoyl peroxide, triethylborane, and diethylzinc. In addition, the polymerization inhibitor is not particularly limited, and those generally used can be suitably used, and examples thereof include hydroquinone, p-methoxyphenol, triphenyl-verdazyl, 2,2,6,6-tetramethylpiperidinyl-1-oxyl, and 4-hydroxy-2,2,6,6-tetramethylpiperidinyl-1-oxyl.

A method of synthesizing a hydrophilic/temperature-responsive polymer in the present invention is not particularly limited, and a living radical polymerization technique described in "Radical Polymerization Handbook," pp. 161 to 225 (2010) published by NTS can be used.

In the present invention, the layer thickness of the layer formed of a temperature-responsive polymer is preferably 1,000 nm or less, more preferably 200 nm or less, particularly preferably 100 nm or less, and most preferably 50 nm or less because it is suitable for improve cell proliferation properties. In addition, the layer thickness of the layer formed of a temperature-responsive polymer is preferably 5 nm or more, more preferably 20 nm or more, particularly preferably 30 nm or more, and most preferably 35 nm or more because it is suitable for rapidly performing a step of forming a cell mass from cell masses.

In the present invention, as necessary, the cell culture substrate may comprise an organism-derived substance on its surface. The organism-derived substance is not particularly limited, and examples thereof include matrigel, laminin, fibronectin, vitronectin, and collagen.

These organism-derived substances may be natural substances or substances artificially synthesized by gene modification technology or the like, or may be fragments cut with a restriction enzyme or the like or synthetic proteins or synthetic peptides based on these organism-derived substances.

In the present invention, regarding the matrigel, commercial products, for example, Matrigel (commercially available from Corning Incorporated) and Geltrex (commercially available from Thermo Fisher Scientific), can be suitably used in consideration of availability.

The type of the laminin is not particularly limited, and for example, laminin 511, laminin 521 or laminin 511-E8 fragments which have been reported to exhibit high activity with respect to α6β1 integrin expressed on the surface of human iPS cells can be used. The laminin may be a natural substance or a substance artificially synthesized by gene modification technology or the like, or synthetic proteins or synthetic peptides based on the laminin. In consideration of availability, commercial products, for example, iMatrix-511 (commercially available from Nippi, Inc.), can be suitably used.

The vitronectin may be a natural substance or a substance artificially synthesized by gene modification technology or the like, or synthetic proteins or synthetic peptides based on the vitronectin. In consideration of availability, commercial products, for example, Vitronectin, from Human Plasma (commercially available from Wako Pure Chemical Industries, Ltd.), synthemax (commercially available from Corning Incorporated), and Vitronectin (VTN-N) (commercially available from Thermo Fisher Scientific), can be suitably used.

The fibronectin may be a natural substance or a substance artificially synthesized by a gene modification technology or the like, or synthetic proteins or synthetic peptides based on the fibronectin. In consideration of availability, commercial products, for example, Fibronectin Solution, from Human Plasma (commercially available from Wako Pure Chemical Industries, Ltd.), and Retronectin (commercially available from Takara Bio Inc.), can be suitably used.

The type of the collagen is not particularly limited, and for example, type I collagen and type IV collagen can be used. The collagen may be a natural substance or a substance artificially synthesized by a gene modification technology or the like, or synthetic peptides based on the collagen. In consideration of availability, commercial products, for example, Collagen I, Human (commercially available from Corning Incorporated) and Collagen IV, Human (commercially available from Corning Incorporated), can be suitably used.

In the present invention, the organism-derived substance is preferably immobilized on the cell culture substrate by a non-covalent bond rather than a covalent bond because it is possible to prevent denaturation of the organism-derived substance and it is possible to improve cell proliferation properties. Here, in the present invention, the "non-covalent bond" indicates a binding force other than a covalent bond derived from an intermolecular force such as an electrostatic interaction, a water-insoluble interaction, a hydrogen bond, a π-π interaction, a dipole-dipole interaction, a London dispersion force, and other Van der Waals interactions. Immobilization of the organism-derived substance on the block copolymer may be due to a single binding force or a combination of a plurality of binding forces.

In the present invention, the method of immobilizing an organism-derived substance is not particularly limited, but for example, a method of applying an organism-derived substance solution to a cell culture substrate for a predetermined time for immobilization, or a method of adding an organism-derived substance to a culture solution when cells are cultured and thus adsorbing the organism-derived substance to the cell culture substrate for immobilization can be suitably used.

In the present invention, the material of the substrate is not particularly limited, and not only substances such as glass and polystyrene generally used for cell culture but also substances that can generally impart forms, for example, polymer compounds such as polycarbonate, polyethylene terephthalate, polyvinylidene fluoride, polytetrafluoroethylene, polyethylene, polypropylene, and polymethylmethacrylate, ceramic, and metals can be used. In consideration of ease of a culture operation, the material of the substrate preferably comprises at least one type of glass, polystyrene, polycarbonate, polyethylene terephthalate, polyethylene, and polypropylene, more preferably comprises at least one type of glass, polystyrene, polycarbonate, polyethylene terephthalate, and polyethylene, and particularly preferably comprises polystyrene, polycarbonate, polyethylene terephthalate, or polyethylene because it is suitable to increase the flexibility. In addition, polystyrene, polycarbonate or polyethylene terephthalate is most preferable because it is suitable to impart cell proliferation properties by patterning according to a hydrophilization treatment to be described below.

The shape of the substrate is not particularly limited, and may be a planar shape such as a plate or a film or may be a fiber, a porous particle, a porous membrane, or a hollow fiber. In addition, a container generally used for cell culture or the like (a cell culture disk such as a petri dish, a flask, a plate, a bag, etc.) may be used. In consideration of ease of a culture operation, a planar shape such as a plate or a film or a flat porous membrane is preferable. In addition, a structure for classifying cell masses may be provided by providing partition plates on the substrate as necessary.

In the present invention, preferably, the substrate is a porous substrate, and the pore diameter of the porous substrate is smaller than that of cells because it is suitable to rapidly perform a step of forming a cell mass from cell masses, and further, even if large cell masses are manufactured, it is possible to uniformly distribute nutrition to the inside of the cell masses. The pore diameter is preferably 0.01 to 8 µm, more preferably 0.01 to 3 µm, particularly preferably 0.01 to 1 µm, and most preferably 0.1 to 1 µm so that it is suitable to rapidly perform a step of forming a cell mass from cell masses. Here, in the present invention, the "pore diameter" of the porous substrate indicates an average value of diameters of pores of the porous substrate in the in-plane direction of the porous substrate and can be calculated by measuring diameters of 20 or more pores in a laser microscopic image, a scanning electron microscopic image, or a transmission electron microscopic image of the porous substrate and obtaining an average value.

The porosity of the porous substrate is preferably 0.01 to 60%, more preferably 0.01 to 20%, particularly preferably 0.01 to 4%, and most preferably 0.01 to 1.5% so that it is suitable to rapidly perform a step of forming a cell mass from cell masses. Here, in the present invention, the "porosity" of the porous substrate indicates a value obtained by dividing a total area of pore parts by a substrate area for one main surface of the surfaces of the porous substrate, and indicates the amount of voids present on the surface of the substrate in terms of area ratio, and this can be measured by observing a square region having one side with a length of 200 times or more the pore diameter of pores of the porous substrate in a laser microscopic image, a scanning electron microscopic image, or a transmission electron microscopic image of the porous substrate.

The cell culture substrate of the present invention may be sterilized. A sterilization method is not particularly limited, and high pressure steam sterilization, UV sterilization, γ-ray sterilization, ethylene oxide gas sterilization, or the like can be used. High pressure steam sterilization, UV sterilization, or ethylene oxide gas sterilization is preferable because it is suitable to prevent denaturation of the block copolymer, UV sterilization or ethylene oxide gas sterilization is more preferable because it is suitable to prevent deformation of the substrate, and ethylene oxide gas sterilization is particularly preferable because it is excellent in mass productivity.

Cells used in the method for manufacturing a cell mass of the present invention are not particularly limited as long as they can adhere to the cell culture substrate. For example, in addition to various strained cells such as Chinese hamster ovary-derived CHO cells, mouse connective tissue L929, human fetal kidney-derived HEK293 cells, and human cervical cancer-derived HeLa cells, for example, epithelial cells and endothelial cells that constitute tissues and organs in living bodies, skeletal muscle cells, smooth muscle cells, and myocardial cells exhibiting contractility, neuron cells, glia cells, and fibroblasts that constitute the nervous system, hepatocytes, hepatic nonparenchymal cells, and adipocytes involved in metabolism of living bodies, and as cells having a differentiation ability, stem cells present in various tissues such as mesenchymal stem cells, bone marrow cells, and Muse cells, and also, stem cells (pluripotent stem cells) having pluripotency such as ES cells and iPS cells, and cells induced to differentiate from them can be used. It is preferable to use epithelial cells and mesenchymal cells because they are suitable to form an organ primordium.

A method for manufacturing a cell mass of the present invention comprises the following steps (1) to (3).
(1) a step of preparing a cell suspension comprising mesenchymal cells and ectoderm-derived cells.
(2) a step of bringing the cell suspension prepared in the step (1) into contact with a cell culture substrate, and randomly adhering the mesenchymal cells and the ectoderm-derived cells to a region (A), and in which, on the contact surface between the cell suspension and the cell culture substrate, a total number of cells of the mesenchymal cells and the ectoderm-derived cells is 100 to 100,000 cells per unit area.
(3) a step of culturing the cells adhered to the region (A) and forming a cell mass comprising the mesenchymal cells and the ectoderm-derived cells.

In addition, the method for manufacturing a cell mass of the present invention may comprise the following step (4).
(4) a step of culturing the cell mass formed in the step (3) and forming an organ primordium.

In the step (1), a cell suspension comprising two types of cells: mesenchymal cells and ectoderm-derived cells, is prepared. The method of preparing a cell suspension is not particularly limited, and two or more types of cells cultured in advance separately are separated and collected in the form of single cells using an enzyme or the like, and mixed in a medium at a desired ratio. The mixing ratio of two or more types of cells is preferably in a range of 0.5 to 1.5 times, more preferably in a range of 0.8 to 1.2 times, particularly preferably in a range of 0.9 to 1.1 times, and most preferably in a range of 0.95 to 1.05 times the cell ratio when all cells are mixed in a uniform ratio according to the number of cells because it is suitable to form a portion having a larger content of one type of cells. Ratio between the number of mesenchymal cells and the number of ectoderm-derived cells in the cell suspension may be 1:10 to 10:1 or 1:5 to 5:1, and is preferably 1:1. When ratio between the number of mesenchymal cells and the number of ectoderm-derived cells in the cell suspension is within these ranges, it is suitable to form a portion having a larger content of mesenchymal cells or ectoderm-derived cells.

In the step (2), the cell suspension prepared in the step (1) is brought into contact with a cell culture substrate, and mesenchymal cells and ectoderm-derived cells are randomly adhered to the region (A). Here, a total number of cells of the mesenchymal cells and the ectoderm-derived cells on the contact surface between the cell suspension and the cell culture substrate is 100 to 100,000 cells per unit area. When cells are adhered, since cell masses are immobilized on the cell culture substrate, it is possible to reduce damage to the cell masses due to shaking during a culture operation and transport. When cells are not adhered, cell masses are easily damaged by colliding with wall surfaces of the cell culture substrate and other cell masses during a culture operation and transport. In order to allow a sufficient amount of cells to adhere to the region (A) and easily form a portion having a larger content of one type of cells, the number of seeded cells per area of the cell culture substrate (a total number of cells of the mesenchymal cells and the ectoderm-derived cells on the contact surface between the cell suspension and the cell culture substrate) is preferably 1,000 cells/cm² or more, more preferably 3,000 cells/cm² or more, particularly preferably 5,000 cells/cm² or more, and most preferably 10,000 cells/cm² or more. In addition, in order to prevent aggregation of cells in a suspended state, the number of seeded cells per area of the cell culture substrate (a total number of cells of the mesenchymal cells and the ectoderm-derived cells on the contact surface between the cell suspension and the cell culture substrate) is preferably 100,000 cells/cm² or less, more preferably 80,000 cells/cm² or less, particularly preferably 50,000 cells/cm² or less, and most preferably 20,000 cells/cm² or less.

In the step (3), cells adhered to the region (A) are cultured to form a cell mass comprising mesenchymal cells and ectoderm-derived cells. In addition, when the cell culture substrate having the configuration of this application is used, a cell mass having at least two portions: a portion having a larger content of ectoderm-derived cells and a portion having a smaller content of the cells (a larger content of mesenchymal cells), is formed. When two or more types of cells are cultured in a region having the area described above, only one type of cells can spontaneously aggregate to form a portion having a larger content of one type of cells.

In the step (3), the cell mass may comprise a portion having a larger content of ectoderm-derived cells than mesenchymal cells (simply referred to as a portion having a larger content of ectoderm-derived cells) so that it is suitable to form an organ primordium. In addition, the cell mass may further comprise a portion having a larger content of mesenchymal cells than ectoderm-derived cells (simply referred to as a portion having a larger content of mesenchymal cells). When the cell mass comprises a portion having a larger content of mesenchymal cells and a portion having a larger content of ectoderm-derived cells, a ratio between the largest cross-sectional area of the portion having a larger content of mesenchymal cells in the substrate in-plane direction and the largest cross-sectional area of a portion having a larger proportion of ectoderm-derived cells in the substrate in-plane direction is preferably 2:1 to 1:20, more preferably 2:1 to 1:10, particularly preferably 1:1 to 1:5, and most preferably 1:1 to 1:2 so that it is suitable to form an organ primordium.

In the step (4), the cell mass formed in the step (3) is cultured to form an organ primordium. In the organ primordium, Versican proteins, which are markers for dermal papilla cells, may be expressed.

The cell mass comprises at least two portions: a portion composed of cells adhered to the region (A) and a substantially spherical portion adhered to the adhered cells, and the diameter of the substantially spherical portion is preferably smaller than the diameter of the region (A). When the cell mass has the above shape, differentiation into organ primordia such as hair follicle primordia is likely to occur.

The type of the medium used in the present invention is not particularly limited, and can be appropriately selected depending on cells to be cultured. For example, when two types, mesenchymal cells and epithelial cells, are used as cells to be cultured, a medium in which DMEM (commercially available from Takara Bio Inc.) and a keratinocyte growth medium (commercially available from Takara Bio Inc.) are mixed at 1:1 is preferable.

When two or more types of cells (for example, mesenchymal cells and ectoderm-derived cells) are co-cultured, a larger content of one type of cells (for example, ectoderm-derived cells) means that, when only specific cells are subjected to a treatment that enables fluorescent labeling (immunostaining) and the cell mass is then observed using a phase contrast microscope and a fluorescence microscope, the proportion of the number of one type of cells (for example, ectoderm-derived cells) with respect to the number of cells comprised in the cross section of the cell mass in a certain region is 50% or more. When two or more types of cells (for example, mesenchymal cells and ectoderm-derived cells) are co-cultured, a smaller content of one type of cells (for example, ectoderm-derived cells) means that the proportion is less than 50%.

Another aspect of the present invention relates to a differentiation induction kit for hair follicle primordia comprising a cell culture substrate having the following two regions (A) and (B). As the cell culture substrate comprised in this kit, the same substrate as the cell culture substrate used in the method for manufacturing a cell mass can be used.
(A) a circular region having cell proliferation properties and an area of 0.001 to 1 mm², and
(B) a region adjacent to the region (A) and having no cell proliferation properties.

The differentiation induction kit for hair follicle primordia may further comprise a medium in which mesenchymal cells and epithelial cells have proliferating properties. As the medium in which mesenchymal cells and epithelial cells have proliferating properties, the same medium as the medium used in the method for manufacturing a cell mass (for example, a medium in which DMEM (commercially available from Takara Bio Inc.) and a keratinocyte growth medium (commercially available from Takara Bio Inc.) are mixed at 1:1) can be used.

### [Examples]

Hereinafter, the present invention will be described in detail with reference to embodiments for implementing the present invention, but these embodiments are only examples for describing the present invention, and the present invention is not limited to the following content. In addition, the embodiments can be appropriately changed and implemented within the scope of the present invention. Here, unless otherwise specified, commercially available reagents were used.

[Example 1] A dish having a diameter of 35 mm and coated with a hydrophilic polymer on the surface (product name: PrimeSurface commercially available from Sumitomo Bakelite Co., Ltd.) was covered with a metal mask with a plurality of circular holes having a diameter of 0.2 mm (commercially available from Mitani Micronics Co., Ltd.), a plasma treatment (under a gas pressure of 20 Pa, a conduction current of 20 mA, for an emission time of 30 seconds) was performed over the metal mask using a plasma emission device (product name: plasma ion bombarder PIB-20 commercially available from Vacuum Device), and a cell culture substrate having a cell-adhesive and cell-proliferative region was prepared.

Mesenchymal cells (mesenchymal stem cells derived from bone marrow) and epithelial cells (human gingival epithelial cells) cultured in a polystyrene flask were separated in a 0.5×TrypLE-EDTA solution and centrifuged, and the cells were dispersed again in a medium in which DMEM (commercially available from Takara Bio Inc.) and a keratinocyte growth medium (commercially available from Takara Bio Inc.) were mixed at 1:1. The number of cells was measured, and a cell suspension in which mesenchymal cells and epithelial cells were mixed at 1:1 was prepared.

The cell suspension was added to the patterned cell culture substrate and cells were seeded at 4,500 cells/cm². The cells were cultured for 12 days in an environment of 37°C and a CO₂ concentration of 5%. According to observation under a phase contrast microscope, it was confirmed that the cells were randomly adhered in the region (A) on the 1st day of culture, epithelial cells aggregated on the 4th day of culture, and a cell mass comprising a portion having a larger proportion of epithelial cells was formed. In addition, the cell mass on the 11th day of culture comprised at least two portions: a portion composed of cells adhered to the region (A) and a substantially spherical portion adhered to the adhered cells.

When the cell culture substrate on which the cell mass was formed was shaken for 1 hour using a shaker, all cells were immobilized in an adhesion state, and damage to the cell mass was not observed.

[Example 2] A metal mask with a plurality of circular holes having a diameter of 0.5 mm was used in place of a metal mask with a plurality of circular holes having a diameter of 0.2 mm in Example 1. In addition, culture was performed at a cell seeding density of 15,000 cells/cm² in place of a cell seeding density of 4,500 cells/cm² in Example 1. The cells were randomly adhered in the region (A) on the 1st day of culture, epithelial cells aggregated on the 6th day of culture, and a cell mass comprising a portion having a larger proportion of epithelial cells was formed.

When the cell culture substrate on which the cell mass was formed was shaken for 1 hour using a shaker, all cells were immobilized in an adhesion state, and damage to the cell mass was not observed.

[Example 3] Culture was performed in the same manner as in Example 1 except that human dermal papilla cells fluorescently stained with Vybrant DiO Cell-Labeling Solution were used as mesenchymal cells.

The cultured cells were observed under a phase contrast microscope and a fluorescence microscope, and it was confirmed that, on the 1st day of culture, mesenchymal cells and epithelial cells were randomly present in the region (A), and on the 7th day of culture, a cell mass comprising a portion having a larger proportion of mesenchymal cells (a portion having a smaller proportion of epithelial cells) and a portion having a smaller proportion of mesenchymal cells (a portion having a larger proportion of epithelial cells) was formed. The cell mass was imaged from the vertical direction of the cell culture substrate by a phase contrast microscope and a fluorescence microscope. In the image, a ratio between the largest cross-sectional area of a portion having a larger content of mesenchymal cells (a portion having a smaller proportion of epithelial cells) in the substrate in-plane direction and the largest cross-sectional area of a portion having a smaller proportion of mesenchymal cells (a portion having a larger proportion of epithelial cells) in the substrate in-plane direction was 1:8.5.

[Example 4] Culture was performed in the same manner as in Example 1 except that human dermal papilla cells were used as mesenchymal cells, and Versican was then fluorescently stained using Human Versican Isoform V0 Antibody (commercially available from R&D SYSTEMS) and Donkey anti-Goat IgG (H+L) Cross-Adsorbed Secondary Antibody, Alexa Fluor 488 (commercially available from Thermo Fisher).

The cultured cells were observed under a phase contrast microscope and a fluorescence microscope, and it was confirmed that, on the 1st day of culture, mesenchymal cells and epithelial cells were randomly present in the region (A), and on the 7th day of culture, a cell mass comprising a portion having a larger proportion of mesenchymal cells (a portion having a smaller proportion of epithelial cells) and a portion having a smaller proportion of mesenchymal cells (a portion having a larger proportion of epithelial cells) was formed. In addition, the cultured cells were observed under a fluorescence microscope, and it was confirmed that Versican was expressed. The cell mass was imaged from the vertical direction of the cell culture substrate by a phase contrast microscope and a fluorescence microscope. In the image, a ratio between the largest cross-sectional area of a portion having a larger content of mesenchymal cells (a portion having a smaller proportion of epithelial cells) in the substrate in-plane direction and the largest cross-sectional area of a portion having a smaller proportion of mesenchymal cells (a portion having a larger proportion of epithelial cells) in the substrate in-plane direction was 1:4.9.

[Example 5] A cell culture substrate having a cell-adhesive and cell-proliferative region was prepared using a metal mask with a plurality of circular holes having a diameter of 0.1 mm in place of a metal mask with a plurality of circular holes having a diameter of 0.2 mm in Example 1. Culture was performed in the same manner as in Example 4 except that human dermal papilla cells were used as mesenchymal cells.

The cultured cells were observed under a phase contrast microscope and a fluorescence microscope, and it was confirmed that, on the 1st day of culture, mesenchymal cells and epithelial cells were randomly present in the region (A), and on the 7th day of culture, a cell mass comprising a portion having a larger proportion of mesenchymal cells (a portion having a smaller proportion of epithelial cells) and a portion having a smaller proportion of mesenchymal cells (a portion having a larger proportion of epithelial cells) was formed. In addition, the cultured cells were observed under a fluorescence microscope, and it was confirmed that Versican was expressed. The cell mass was imaged from the vertical direction of the cell culture substrate by a phase contrast microscope and a fluorescence microscope. In the image, a ratio between the largest cross-sectional area of a portion having a larger content of mesenchymal cells (a portion having a smaller proportion of epithelial cells) in the substrate in-plane direction and the largest cross-sectional area of a portion having a smaller proportion of mesenchymal cells (a portion having a larger proportion of epithelial cells) in the substrate in-plane direction was 1.5:1.

[Comparative Example 1] Culture was performed in the same manner as in Example 1 except that only epithelial cells were cultured without using mesenchymal cells in Example 1. Although culture was continued for 12 days, only cell masses in which all cells were uniformly distributed were formed, and cell masses comprising a portion having a larger proportion of one type of cells were not formed.

[Comparative Example 2] Culture was performed in the same manner as in Example 1 except that only mesenchymal cells were cultured without using epithelial cells in Example 1. Although culture was continued for 12 days, only cell masses in which all cells were uniformly distributed were formed, and cell masses comprising a portion having a larger proportion of one type of cells were not formed.

[Comparative Example 3] A metal mask with a plurality of circular holes having a diameter of 1.5 mm was used in place of a metal mask with a plurality of circular holes having a diameter of 0.2 mm in Example 1. In addition, culture was performed at a cell seeding density of 15,000 cells/cm² in place of a cell seeding density of 4,500 cells/cm² in Example 1. Although culture was continued for 12 days, only cell masses in which all cells were uniformly distributed were formed, and cell masses comprising a portion having a larger proportion of one type of cells were not formed.

[Comparative Example 4] Culture was performed in the same manner as in Example 1 except that a cell-non-adhesive U bottom container (product name: PrimeSurface 96-well plate commercially available from Sumitomo Bakelite Co., Ltd.) was used as the cell culture substrate, and the cell seeding density was 2,400 cells/well. It was confirmed that, on the 4th day of culture, epithelial cells aggregated, and a cell mass comprising a portion having a larger proportion of epithelial cells was formed.

When all the cell masses were collected in a dish having a diameter of 35 mm and whose surface was coated with a hydrophilic polymer (product name: PrimeSurface commercially available from Sumitomo Bakelite Co., Ltd.), and the cell culture substrate was shaken for 1 hour using a shaker, the cell mass was damaged, and did not retain its original shape.

[Comparative Example 5] Culture was performed at a cell seeding density of 150,000 cells/cm² in place of a cell seeding density of 4,500 cells/cm² in Example 1. On the 7th day of culture, a cell mass was formed in the condition that the cells were suspended. When the cell culture substrate was shaken for 1 hour using a shaker, the cell mass was damaged, and did not retain its original shape.

**[Table 1]**

| | Characteristics of cell culture substrate | | Step (1) | Step (2) | Step (3) | | Evaluation |
|---|---|---|---|---|---|---|---|
| | Shape of region (A) | Area of region (A) (mm²) | Ratio between number of mesenchymal cells and number of epithelial cells | Total number of cells per substrate unit area (cells/cm²) | Formation of portion having larger proportion of mesenchymal cell and portion of having larger proportion of epithelial cells | Ratio between largest cross-sectional area of portion having larger content of mesenchymal cells and largest cross-sectional area of portion having smaller proportion of mesenchymal cells | Condition of cell mass after culture substrate was shaken |
| Example 1 | Diameter of 0.2 mm, circle | 0.03 | 1:1 | 4500 | Yes | - | No damage |
| Example 2 | Diameter of 0.5 mm, circle | 0.20 | 1:1 | 15000 | Yes | - | No damage |
| Example 3 | Diameter of 0.2 mm, circle | 0.03 | 1:1 | 4500 | Yes | 1:8.5 | - |
| Example 4 | Diameter of 0.2 mm, circle | 0.03 | 1:1 | 4500 | Yes | 1:4.9 | - |
| Example 5 | Diameter of 0.1 mm, circle | 0.01 | 1:1 | 4500 | Yes | 1.5:1 | - |
| Comparative Example 1 | Diameter of 0.2 mm, circle | 0.03 | 0:1 | 4500 | No | - | - |
| Comparative Example 2 | Diameter of 0.2 mm, circle | 0.03 | 1:0 | 4500 | No | - | - |
| Comparative Example 3 | Diameter of 1.5 mm, circle | 7.07 | 1:1 | 4500 | No | - | - |
| Comparative Example 4 | Without region (A) | 0 | 1:1 | 2400 cells/well | Yes | - | Damage |
| Comparative Example 5 | Diameter of 0.2 mm, circle | 0.03 | 1:1 | 150000 | No | - | Damage |

### Reference Signs List

A Region (A)
B Region (B)
1 Epithelial cell
2 Mesenchymal cell
3 Cell mass
4 Cell mass comprising at least two portions: portion having larger content of epithelial cells (smaller content of mesenchymal cells) and portion having smaller content of epithelial cells (larger content of mesenchymal cells)

## Claims

1. A method for manufacturing a cell mass adherently cultured on a cell culture substrate in which the cell culture substrate comprises the following two regions (A) and (B), and the method comprising the following steps (1) to (3):
(A) an island-shaped region having a cell proliferation property and an area of 0.001 to 1 mm²; and
(B) a region adjacent to the region (A) and having no cell proliferation properties,
(1) a step of preparing a cell suspension comprising a mesenchymal cell and an ectoderm-derived cell;
(2) a step of bringing the cell suspension prepared in the step (1) into contact with the cell culture substrate, and randomly adhering the mesenchymal cell and the ectoderm-derived cell to the region (A), and in which, on the contact surface between the cell suspension and the cell culture substrate, a total number of cells of the mesenchymal cell and the ectoderm-derived cell is 100 to 100,000 cells per unit area; and
(3) a step of culturing the cell adhered to the region (A) and forming a cell mass comprising the mesenchymal cell and the ectoderm-derived cell.

2. The method for manufacturing a cell mass according to claim 1,
wherein a ratio between the number of mesenchymal cell and the number of ectoderm-derived cell in the cell suspension is 1:10 to 10:1.

3. The method for manufacturing a cell mass according to claim 1 or 2,
wherein, in the step (3), the cell mass comprises a portion having a larger content of the mesenchymal cell than the ectoderm-derived cell and a portion having a larger content of the ectoderm-derived cell than the mesenchymal cell, and
wherein a ratio between the largest cross-sectional area of a portion having a larger content of the mesenchymal cell than the ectoderm-derived cell in the substrate in-plane direction and the largest cross-sectional area of a portion having a larger content of the ectoderm-derived cell than the mesenchymal cell in the substrate in-plane direction is 2:1 to 1:20.

4. The method for manufacturing a cell mass according to any one of claims 1 to 3, further comprising the following step (4): (4) a step of culturing the cell mass formed in the step (3) and forming an organ primordium.

5. The method for manufacturing a cell mass according to claim 4,
wherein Versican is present in the organ primordium.

6. A differentiation induction kit for hair follicle primordia comprising a cell culture substrate having the following two regions (A) and (B):
(A) a circular region having a cell proliferation property and an area of 0.001 to 1 mm², and
(B) a region adjacent to the region (A) and having no cell proliferation properties.

7. The differentiation induction kit for hair follicle primordia according to claim 6, further comprising a medium in which a mesenchymal cell and an epithelial cell have proliferating properties.
